# EUROPEAN PATENT SPECIFICATION

(11) **EP 3 000 459 B1**
(45) Date of publication and mention of the grant of the patent: **17.07.2019**
(21) Application number: 15187471.6
(22) Date of filing: 29.09.2015
(51) Int. Cl.: A61K 8/25, A61K 8/27, A61K 8/365, A61K 8/46, A61Q 15/00, A61K 8/58, A61K 8/73, A61K 8/81

(54) **ANTIPERSPIRANT COMPOSITIONS**
SCHWEISSHEMMENDE ZUSAMMENSETZUNGEN
COMPOSITIONS ANTI-TRANSPIRANTES

(30) Priority: 29.09.2014 US 201414499713
(43) Date of publication of application: 30.03.2016
(73) Proprietor: Johnson & Johnson Consumer Inc., Skillman, NJ 08558 (US)
(72) Inventor: ANCONI, Glasiela Lemos, 12241-070 Sao Paulo (BR); LOFFREDO, Luciana De Castro Monteiro, 12241-070 Sao Paulo (BR); PASSERO, Alan, 12240-907 Sao Paulo (BR); SHERGUE, Ellen Muniz, 02436 000 Sao Paulo (BR)
(74) Representative: Carpmaels & Ransford LLP

(56) References cited:
- WO-A1-93/01793
- WO-A1-97/19674
- WO-A1-98/56340
- US-A- 5 861 143
- US-A- 5 861 146

## Description

### Field of the Invention

The present invention provides an antiperspirant composition comprising at least 5 % by weight of zinc phenolsulphonate or and/or at least 3.5% by weight of zinc lactate, wherein said composition is substantially free of aluminum salts.

### Background of the Invention

Antiperspirant and deodorant compositions are widely employed throughout many parts of the world in order to control localized perspiration and odor. Wet patches such as those under the arms are considered to be unsightly. Additionally, body odor can interfere with normal relationships and cause social exclusion. These issues take on a greater significance particularly in areas that tend to have high humidity such as the warmer climates. Besides the underarm area, other body regions including the feet may benefit from the use of antiperspirants and deodorants.

Antiperspirants act by reducing the sweating process. This may be accomplished by clogging the pore from which the sweat is produced, blocking the acetylcholine receptor of the sweat gland, or inhibiting the production of the sweat at the gland (prevention of the release of acetylcholine from sympathic neurons). Deodorants on the other hand act by stopping the sweat from smelling. Deodorant agents do this by limiting bacterial proliferation, blocking sweat diffusion, absorbing malodors and enzymatic inhibition. Commercial products may be marketed as a deodorant, an antiperspirant, or both.

Products currently marketed for such use may employ an astringent metal salt, such as an aluminium or aluminium/zirconium salt. Such salts are not only capable of blocking pores to inhibit and reduce sweating, but are also microbicidal and a significant fraction of the salts typically remain on the skin surface between pores. By controlling perspiration with such salts, the user advantageously can simultaneously reduce or eliminate malodour generated locally. Accordingly, if insufficient antiperspirant active is present to enable the composition to function as an effective antiperspirant, the active normally provides deodorant benefits.
Recently, concerns have been raised that aluminum can be absorbed into the bloodstream. Additionally, use of an aerosol antiperspirant containing aluminum allows for inhalation and may result absorption of aluminum from the nasal recess.

Applicant has now advantageously discovered that aluminum salt-free antiperspirants may be formulated using zinc salts in certain amounts. Zinc salts have been described in literature as deodorant agents. However, recommended concentrations when used as deodorants may vary only up to about 2.0 w/w% for some zinc salts.

It is known that aluminum salts operate by precipitation of proteins of luminal epithelial cells, such as collagen, keratin, glycoproteins and others which clog the sweat gland. When used according to the invention, zinc lactate and zinc phenolsulfonate also demonstrate antiperspirant effects related to sweat control. In order to accomplish an antiperspirant effect, higher concentrations of the zinc salts are required. Similar properties are expected for zinc lactate and zinc phenolsulfonate due to the potential of the organic chain to polymerize. Such a characteristic is not expected for inorganic zinc salts, such as zinc oxide, due to the absence of carbon chain and having little possibility of polymerization.

### Summary of the Invention

The present invention provides an antiperspirant composition comprising at least 5 % by weight of zinc phenolsulphonate and/or at least 3.5% by weight of zinc lactate, wherein said composition is substantially free of aluminum salts.

### Detailed Description of the Invention

The present invention relates to the use of an effective amount of a zinc salt sufficient to act as an antiperspirant.

The antiperspirant may include other ingredients such as water absorbing agents.

By combining an effective amount of a zinc salt with an absorbing agent, an effective and aesthetically pleasing antiperspirant formulation may be attained.

As used herein, the term "effective amount of a zinc salt" means an amount to sufficiently block or clog a sweat gland from producing sweat on the surface of the skin. The skin remains relatively dry when the seat gland is blocked or clogged. The amount required to be effective as an antiperspirant is more than what is normally used in a deodorant.

In particular, the composition comprises at least 3.5% by weight of zinc salt. The amount of zinc salt is substantially higher than previously known for deodorant protection; this higher amount provides antiperspirant protection.

Preferably, for zinc lactate, the composition comprises at least 3.5% by weight, more preferably at least 3.6% by weight zinc salt.

Preferably, for zinc phenolsulphonate, the composition comprises at least 5% by weight, more preferably at least 5.3% by weight zinc salt.

In one embodiment, the zinc salt is zinc phenolsulphonate.

In another embodiment, the zinc salt is zinc lactate.

A mixture of both zinc phenolsulphonate and zinc lactate may also be used.

The composition may comprise one or more additional antiperspirant actives, for example Sodium Lactate, Calcium Lactate, Ammonium Lactate, Calcium Acetate, Sodium Acetate and Magnesium Acetate.

Antiperspirant compositions according to the invention may generally contain up to 20%, or up to about 13%, or up to about 10%, by weight zinc salt plus any additional antiperspirant actives.

The composition is substantially free of aluminum chlorohydrate and/or its derivatives. The formulations may occasionally contain aluminum residue. This is related to traces or extremely low amounts of aluminum element as a residue in raw materials used as ingredients of the composition. This is not an aluminum salt.

The composition may optionally comprise a water absorbing agent. Such water absorbing agents are known for cosmetic applications and are usually porous ingredients, composed of micro channels resulting in a high absorption capacity and the ability to absorb a different variety of materials. Such compounds may have affinity to hydrophilic or lipophilic molecules. In some cases, it is preferred to combine multiple water absorbing agents in order to maximize the absorption capabilities.

Suitable water absorbers include silicas, cotton powder, bamboo silk and zeolites.

In one embodiment, the composition comprises Bambusa Arundinacea Stem Powder (for example BAMBOO SILK PW available from Polytechno Indústrias Químicas Ltda/Ion Química), and silicas (for example MSS 500/3H available from Kobo and/or SPHERON L-1500 available from Presperse).

The composition may comprise other ingredients known in the antiperspirant, deodorant, or cosmetic art. In most embodiments, the antiperspirant will include other functional ingredients, which provide benefits to the user's body. Such materials are in general well-known to those persons of ordinary skill in the relevant personal care composition art, and may include moisturizing agents, anti-bacterial agents, deodorants and perfumes.

Other ingredients may include film formers. For example, water-insoluble films of polymers create occlusion on the axillary skin, thereby reducing the underarm wetness. Such films are a barrier to the passage of sweat. Polymers used should be occlusive, insoluble in water, and high in degree of adhesion, such as Acrylates/Octylacrylamide Copolymer.

The composition may also include ingredients known to have antibacterial and deodorant properties. These substances may include, for example, 1,6-di-(4-chlorophenylbiguanido) hexane (Chlorhexidin), 3,4,4'-trichlorocarbanilide, quaternary ammonium compounds, oil of cloves, mint oil, thyme oil, triethyl citrate, farnesol (3,7,11-trimethyl-2,6,10-dodecatrien-1-ol), ethylhexyl glycerol ether, or polyglyceryl-3 caprylate. In particular, polyglyceryl 3 caprylate (TEGO® Cosmo P 813, available from Degussa/Evonik), a vegetable sourced ester, with lipophilic character, has been used to reduce odor-causing bacteria on the skin at very low concentrations.

The composition may also comprise linear chain alcohols, amines, ether-alcohols, cetyl alcohol, stearyl alcohol and cetearyl alcohol, classified by CTFA as "emulsion stabilizers" agents.

Perfumes and fragrances may be included in the antiperspirant composition. Perfumes give rise to an olfactory response in the form of a fragrance, and in addition may contribute to mask body malodor.

Preservatives are required to prevent product damage caused by bacteria, yeast or mold, and to protect the product from inadvertent contamination by the consumer during use. Useful preservatives include sodium benzoate, ethylhexylglycerin and caprylyl glycol, benzyl alcohol, methyl paraben, propyl paraben, DMDM hydantoin, methylchloroisothiaoline, methylisothiazolinone, imidazolidinyl urea phenoxyethanol, sodium benzoate and benzoic acid. EDTA and salts thereof are often used to further enhance preservation.

Antioxidants should be added to prevent formula from oxidation process. Useful antioxidants include ascorbic acid, tocopheryl acetate and polypnehols. In the present invention, anti-oxidants such as tocopheryl acetate (stabilized vitamin E) may be used as anti-oxidant agent.

Humectants and moisturizing agents may be included in the composition. Suitable ingredients include hydrophobic agents, hydrophilic agents and combinations thereof. Examples of moisturizing agents are allantoin, glycerol, polyglycerylmethacrylate, panthenol, polyols, ceramide, borage oil (linoleic acid), hyaluronic acid, sodium peroxylinecarbolic acid (sodium PCA), wheat protein (e.g., laurdimonium hydroxypropyl hydrolyzed wheat protein), hair keratin amino acids, panthenol; primrose oil; GLA 3 and other fish oils that may include, for example, the omega-3 and omega-6 oils and/or linoleic acid; and flax seed oil, and mixtures thereof. Other moisturizing agents can also be used.

Pigments and colorants may be included in the antiperspirant composition of the present invention.

Emollients, such as isopropylmyristate, mineral oils and vegetable oils in general, which give rise to a tactile response in the form of an increase in skin lubricity may be included for a more esthetically pleasing product. The composition may contain emollients in any desired amount to achieve a desired emollient effect.

Non-volatile emollients are preferable in the present invention. Classes of non-volatile emollients include non-silicone and silicone emollients. Non-volatile, non-silicone emollients include C₁₂₋₁₅ alkyl benzoate. The non-volatile silicone material can be a polyethersiloxane, polyalkyarylsiloxane or polyethersiloxane copolymer. An illustrative non-volatile silicone material in the present invention is phenyl trimethicone. Non-limiting examples of emollients can be found in U.S. Pat. No. 6,007,799. Examples include, but are not limited to, PPG-14 butyl ether, PPG-15 stearyl ether, PPG-3 myristyl ether, stearyl alcohol, stearic acid, glyceryl monoricinoleate, isobutyl palmitate, glyceryl monostearate, isocetyl stearate, sulphated tallow, oleyl alcohol, propylene glycol, isopropyl laurate, mink oil, sorbitan stearate, cetyl alcohol, hydrogenated castor oil, stearyl stearate, hydrogenated soy glycerides, isopropyl isostearate, hexyl laurate, dimethyl brassylate, decyl oleate, diisopropyl adipate, n-dibutyl sebacate, diisopropyl sebacate, 2-ethyl hexyl palmitate, isononyl isononanoate, isodecyl isononanoate, isotridecyl isononanoate, 2-ethyl hexyl palmitate, 2-ethyl hexyl stearate, Di-(2-ethyl hexyl) adipate), Di-(2-ethyl hexyl) succinate, isopropyl myristate, isopropyl palmitate, isopropyl stearate, octacosanol, butyl stearate, glyceryl monostearate, polyethylene glycols, oleic acid, triethylene glycol, lanolin, castor oil, acetylated lanolin alcohols, acetylated lanolin, petrolatum, isopropyl ester of lanolin, fatty acids, mineral oils, butyl myristate, isostearic acid, palmitic acid, PEG-23 oleyl ether, olelyl oleate, isopropyl linoleate, cetyl lactate, lauryl lactate, myristyl lactate, quaternised hydroxy alkyl, aminogluconate, vegetable oils, isodecyl oleate, isostearyl neopentanoate, myristyl myristate, oleyl ethoxy myristate, diglycol stearate, ethylene glycol monostearate, myristyl stearate, isopropyl lanolate, paraffin waxes, glycyrrhizic acid, hydrocyethyl stearate amide.

In one embodiment, the emollient is selected from linear silicones, cyclic silicones, hydrocarbons, polyhydroxy alcohols having more than 3 carbon atoms, liquid or solid polyalkyleneglycol ethers containing a polypropylene glycol (PPG) moiety and terminating in an alkyl ether, and combinations thereof. In another embodiment, the emollient is a volatile silicone having a flash point of 100° C. or less, such as cyclomethicone or trisiloxane. In another embodiment, the emollient is a nonvolatile silicone, such as dimethiconol or a longer chain dimethicone.

Surfactants and emulsifiers may be used in the antiperspirant compositions of the invention. Typical surfactants are disclosed in US2003/0007939A1. Emulsifiers useful in the composition include nonionic, anionic, cationic, amphoteric or zwitterionic and blends thereof. Suitable emulsifiers are disclosed in U.S. Pat. No. 3,755,560 and U.S. Pat. No. 4,421,769. Examples are polyethylene glycol 20, sorbitan monolaurate (Polysorbate 20), polyethylene glycol 20 stearyl ether (Brij 78, Steareth 20), polyethylene glycol ether of lauryl alcohol (Laureth 23), polysorbate 80 (Tween 80), and lecithin.

The composition may be in the form of a solid or gel stick, cream, lotion, spray, squeeze product, or the like, as known in the art.

Accordingly, the zinc salt and other active agents may be contained in a vehicle, as known in the art. The major types of antiperspirant vehicles most frequently fall into the following categories: (a) solutions; (b) emulsions, both oil-in-water and water-in-oil; and including lotions and creams; (c) suspensions; (d) gels; and (e) solids and semi-solids including stick products. Antiperspirant products in some vehicles, including liquids, gels, suspensions and emulsions, can be provided for application via roll-on applicator, as known in the art.

Examples of solvents, in addition to water, that are frequently used in personal care compositions are polypropylene glycol, polyethylene glycol, ethanol, glycerol, ethylene glycol, 1,2,4-butanetriol, 1,2,6-hexanetriol, ethanol, isopropanol, butanetriol, sorbitol esters, butanediol, butylene glycol, hexylene glycol, methylpropanediol, pyrrolidone, N-methylpyrrolidone, dimethyl sulfoxide, dimethyl sulfone and similar solvents and mixtures thereof.

The composition of the invention may be a squeeze product, including hydroalcoholic squeeze products.

The antiperspirant composition can be in gel form. A "gel" in accordance with the present invention is a colloid in which the disperse phase has combined with the continuous phase to produce a viscous, jelly-like product. Gels in accordance with the present invention can be aqueous or nonaqueous. The gels will typically comprise a vehicle comprising a gelling agent such as described above. The vehicle of the gels will also typically comprise a solvent. A silicone surfactant may also be included required to achieve stability.

Emulsions, such as lotions and creams, of the oil-in-water type and water-in-oil type are well-known in the cosmetic art and are useful in the subject invention. Triphase emulstions such as water-in-oil-in-water type may also be used. Oils useful in both types of emulsions, and also for solvents in solvent-based vehicles in general, include hydrocarbon oils and waxes (e.g., petrolatum, mineral oil, micro-crystalline waxes, polyalkenes, paraffins, cerasin, ozokerite, polyethylene, perhydrosqualene, polyalphaolefins, hydrogenated polyisobutenes and combinations thereof). Preferred are fatty acid derivatives, cholesterol, cholesterol derivatives, diglycerides and triglycerides (e.g., castor oil, soy bean oil, derivatized soybean oils such as maleated soy bean oil, safflower oil, cotton seed oil, corn oil, walnut oil, peanut oil, olive oil, cod liver oil, almond oil, avocado oil, palm oil, sesame oil, vegetable oils and vegetable oil derivatives, sunflower seed oil, coconut oil and derivatized coconut oil, cottonseed oil and derivatized cottonseed oil, jojoba oil, cocoa butter and combinations thereof, as well as any of the aforementioned oils that have been partially or fully hydrogenated), acetoglyceride esters (e.g., acetylated monoglycerides), alkyl esters, alkenyl esters (e.g., oleyl myristate, oleyl stearate, oleyl oleate, and combinations thereof), lanolin and its derivatives (e.g., lanolin, lanolin oil, lanolin wax, lanolin alcohols, lanolin fatty acids, isopropyl lanolate, acetylated lanolin, acetylated lanolin alcohols, lanolin alcohol linoleate, lanolin alcohol ricinoleate, hydroxylated lanolin, hydrogenated lanolin and combinations thereof), wax esters (e.g., beeswax and beeswax derivatives, spermaceti, myristyl myristate, stearyl stearate and combinations thereof), sterols and phospholipids, and combinations thereof. Examples of alkyl esters include isopropyl esters of fatty acids and long chain esters of long chain fatty acids, e.g., SEFA (sucrose esters of fatty acids), pentaerythritol esters, aromatic mono, di or triesters, cetyl ricinoleate, isopropyl palmitate, isopropyl myristate, cetyl ricinoleate and stearyl ricinoleate. Other examples include hexyl laurate, isohexyl laurate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, acyl isononanoate lauryl lactate, myristyl lactate, cetyl lactate, and combinations thereof. Still other suitable oils include milk triglycerides (e.g., hydroxylated milk glyceride) and polyol fatty acid polyesters. Also useful are vegetable waxes such as carnauba and candelilla waxes; sterols such as cholesterol, cholesterol fatty acid esters; and phospholipids such as lecithin and derivatives, sphingo lipids, ceramides, glycosphingo lipids, and combinations thereof.

The water-based roll-on is usually an oil-in-water emulsion rather than a water-in-oil system due to the poorer efficiency of the latter. The oil-in-water emulsion presents the active ingredient in a readily accessible solution form in the external phase. Since the active ingredient ends up in the dissolved state, the formulator can use either a liquid or solid antiperspirant active.

The composition of the invention may also be a clear water-in-oil roll-on. These compositions are relatively new on the market. They demonstrate superior aesthetics and leave no residue or deposit on the skin after application. Clarity is achieved simply by following the room temperature order of addition specified.

The composition may alternatively be an aerosol. Sprays or aerosols have the advantage of eliminating direct contact with hands for application. The propellant gas may be, for example, a hydrocarbon (propane, isobutene), a chlorofluorocarbon, carbon dioxide or nitrous oxide.

The composition of the invention may be a suspension.

In one embodiment, the composition is a lotion or a cream.

In another embodiment, the composition is a solid or semi-solid or stick, and comprises an effective amount of at least one active personal care ingredient and a solidifying agent, wherein the composition is in the form of a solid or semi-solid at ambient temperature (e.g., 25° C. and below). Accordingly, the composition may comprise for example mixtures of waxes and oils and solutions based on aqueous, propylene glycol and/or alcohol mixtures solidified for example by sodium stearate. Preferably, the solidifying agent is selected from the group consisting of a wax and sodium stearate.

Solid personal care products of the invention may also be finely divided and used in the form of powders.

Almost all antiperspirant solids employ stearyl alcohol as the gelling agent. Emollients are often added to impart a softer feel and to add glideability. Most solids also contain talc and/or silica. Silica is an effective suspending agent that helps to keep the active ingredient homogeneously suspended throughout the stick. Miscellaneous ingredients often include colours, titanium dioxide (opacifying agent) and allantoin (soothing agent).

Sticks and solids are really complex chemical systems requiring a balance of ingredients designed to make the most of several performance factors including payout, slip or lubricity, chemical stability, softening temperature, and of course, efficacy.

The composition of the invention may also be a foam. Accordingly, the liquid vehicles described above may contain dispersions of gas in the liquid phase. The gas globules may be of any size, from colloidal to macroscopic, as in soap bubbles. Typical liquid foams are those used in shaving creams, etc.

### EXAMPLES

### Example 1

The following methods were used to compare several compounds, including zinc phenolsulfonate, zinc lactate pharma grade and zinc lactate food grade, with aluminum sesquichlorohydrate for antiperspirant activity *in vitro.*

### Method 1: Turbidity measurement caused by gel polymerization in pH alkaline.

In this test, a Hach model 2100 lab turbidimeter was used. One solution containing sodium hydroxide at pH 8.5 was prepared and each compound was tested at 10% concentration after mixing. Turbidity caused by gel polymerization in the alkaline solution was measured using the turbidimetry technique. Gel polymerization indicates antiperspirant activity. The insoluble gel formed should act as an obstruction at the orifice of the sweat gland, reducing the flow of axillary perspiration.

The results are shown in Table 1.

**TABLE 1: Mean of turbidity after addition of compounds at 10% in solution**

| **Sample** | **Turbidity pH 8.5 (NTU)** | **Physical Observation** |
|---|---|---|
| Aluminium Sesquichlorohydrate | 593.0 | Gel Polymerization |
| Zinc Lactate (pharma) | 538.0 | Gel Polymerization |
| Zinc Lactate (food) | 525.5 | Gel Polymerization |
| Zinc Phenolsulfonate | 1.8 | Liquid |
| Sodium lactate | 0.5 | Liquid |
| Magnesium sulfate 7.H2O | 1.5 | Liquid |
| Magnesium sulfate 1.H2O | 0.9 | Liquid |
| Ammonium sulfate | 1.1 | Liquid |
| Magnesium sulfate 5.H2O | 1.5 | Liquid |
| Sodium sulfate | 1.8 | Liquid |
| Calcium lactate | 0.9 | Liquid |
| Magnesium sulfate Andros | 0.5 | Liquid |
| Ammonium lactate | 1.2 | Liquid |
| Sodium acetate | 0.9 | Liquid |
| Calcium acetate | 20.0 | Liquid |
| Magnesium chlorate | 0.4 | Liquid |
| Buffer (CONTROL) | 0.0 | Liquid |

The results presented in Table 1 show that both food and pharma grade zinc lactate had a high potential to gel polymerize in an alkaline environment, and behave similarly to aluminum salts.

### Method 2: In vitro study to evaluate active-protein interaction

The Bradford method (Bradford, M.M., A rapid and sensitive for the quantitation of microgram quantitites of protein utilizing the principle of protein-dye binding. Analytical Biochemistry 72: 248-254. 1976) was used to measure the amount of precipitated protein generated by various compounds including zinc lactate and zinc phenolsulfonate. Bovine serum albumin (BSA) was used as the reference protein to generate the calibration curve. The linear concentration range was determined to be 0.1-1.5mg/mL of protein. The BSA granular, the BSA solution standard and Bradford reagent were all obtained from Sigma-Aldrich (Catalog Numeber: A7906, PO834, B6916). The buffer solution was made by adding 875mL of 0.1 Molar sodium hydroxide (SpecSol) to 125mL 0.1 Molar potassium dihydrogen phosphate (Merck) and its pH was adjusted to BSA isoelectric point (pH 4.9).

Solutions of the different compounds at 10% (m/v) concentration were first tested, followed by concentrations ranging from 1% to 15% (m/v) to establish a dose-response curve.

### Procedure:

1. Prepare buffer by adding 875mL of 0.1 Molar sodium hydroxide to 125mL of 0.1 Molar potassium dihydrogen phosphate, adjusted to pH 4.9. This is the buffer solution used to make the active sample solution, the dose-response curve and the protein solution.
2. To prepare an active sample solution for initial screening, add 5g of the active material to 50mL of the buffer solution. To establish a dose-response curve, add 0.5, 2.5, 5.0 and 7.5g (1%, 5%, 10% and 15% w/v solutions) to 50mL of the buffer solution.
3. To prepare the protein solution, add 4g of albumin (available from Sigma-Aldrich) from bovine serum (BSA) to 2000mL of buffer solution.
4. To prepare the reaction solution, add 10mL of the sample solution (step 2) to 50mL of the protein solution.
5. To prepare the analytical solution, after filtration through a 0.45µm membrane, 0.01mL of reaction solution (step 4) to 3mL of the Bradford reagent. Measure and record the absorbance at 595nm. Determine the protein concentration by comparison to the standard curve.

The results are shown in Tables 2 and 3.

**TABLE 2: Results obtained by screening test (10% active solution)**

| **Sample** | **Precipitation (BSA)** |
|---|---|
| Aluminium Sesquichlorohydrate | 100% |
| Zinc Lactate (pharma) | 31% |
| Zinc Phenolsulfonate | 18% |
| Sodium lactate | 13% |
| Magnesium sulfate 7.H2O | 12% |
| Magnesium sulfate 1.H2O | 11% |
| Ammonium sulfate | 10% |
| Magnesium sulfate 5.H2O | 10% |
| Sodium sulfate | 9% |
| Calcium lactate | 8% |
| Magnesium sulfate Andros | 8% |
| Ammonium lactate | 10% |
| Sodium acetate | 5% |
| Calcium acetate | 5% |
| Zinc Lactate (food) | 5% |
| Magnesium chlorate | 4% |
| Buffer + BSA (CONTROL) | 0% |

**TABLE 3: Results obtained by dose-response assay**

| **Sample Name** | **% BSA Precipitation** |
|---|---|
| Zinc phenolsulfonate (1%) | 5% |
| Zinc phenolsulfonate (5%) | -1% |
| Zinc phenolsulfonate (10%) | 21% |
| Zinc phenolsulfonate (15%) | 31% |
| Zinc Lactate - pharma (1%) | 9% |
| Zinc Lactate - pharma (5%) | 7% |
| Zinc Lactate - pharma (10%) | 13% |
| Zinc Lactate - pharma (15%) | 18% |
| Aluminum chlorohydrate (1%) | 24% |
| Aluminum chlorohydrate (5%) | 56% |
| Aluminum chlorohydrate (10%) | 79% |
| Aluminum chlorohydrate (15%) | 95% |
| Buffer pH4.9 + BSA (CONTROL) | 0% |

The results shown in Tables 2 and 3 indicate that increasing the amount of the zinc salts (zinc lactate and zinc phenolsulfonate) exhibit a dose-response correlation, given that precipitation increases with increasing concentration. Based on the results above, zinc lactate pharma grade and zinc phenolsulfonate demonstrated potential to be clogging agents.

### Method 3: Scanning Electron Microscopy (SEM) and pig ear skin (ex-vivo)

Scanning electron microscopy (SEM) was used as a complementary assay to investigate to the obstruction of sweat glands using pig ear skin. SEM photographs of pig ear skin samples taken before and after treatment with different test compositions were prepared and compared as follows.

Pig skin samples were obtained from just below the ear. Untreated skin samples (negative control) were compared with skin samples treated with the aluminum chlorohydrate formulation shown in Table 4 (positive control) or the zinc lactate formulation shown in Table 5. Prior to treatment, the skin samples were dehydrated by washing in sequence with ethanol 70%, ethanol 96% and isopropanol 100%. The skin samples were kept in desiccators 3 days. Prior to treatment, the skin samples were coated with 15 nm platinum.

**TABLE 4**

| INGREDIENT | FUNCTION | % w/w |
|---|---|---|
| A: Aqua | Solvent | Qsp |
| B: Steareth-2 | Surfactant | 1.00-5.00 |
| C: Steareth-21 | Surfactant | 1.00-5.00 |
| D: PPG-15 Stearyl Ether | Emollient | 1.00-10.00 |
| E: Aluminum Chlorohydrate Solution (50%) | Antiperspirant Agent | 12.00-40.00 |
| F: Aluminum Starch Octenylsuccinate | Absorbent | 0.1-3.0 |
| G: Silica | Absorbent | 0.1-2.0 |
| H: Ethylhexyglycerin; Caprylyl Glycol | Skin Conditioning Agent | 0.5-2.0 |
| I: Polyglyceryl-3 Caprylate | Emulsifying Agent | 0.1-1.0 |
| J: Tocopheryl Acetate | Skin Conditioning Agent | 0.01-1.0 |
| K: Sodium Benzoate | Preservative | 0.1-0.6 |

**TABLE 5**

| INGREDIENT | FUNCTION | % w/w |
|---|---|---|
| A: Aqua | Solvent | Qsp |
| B: Steareth-2 | Surfactant | 1.00-5.00 |
| C: Steareth-21 | Surfactant | 1.00-5.00 |
| D: PPG-15 Stearyl Ether | Emollient | 1.00-10.00 |
| E: Zinc Lactate (Pharma) | Antiperspirant Agent | 5.00-20.00 |
| F: Silica | Absorbent | 0.1-2.0 |
| G: Ethylhexyglycerin; Caprylyl Glycol | Skin Conditioning Agent | 0.5-2.0 |
| H: Polyglyceryl-3 Caprylate | Emulsifying Agent | 0.1-1.0 |
| I: Tocopheryl Acetate | Skin Conditioning Agent | 0.01-1.0 |
| J: Sodium Benzoate | Preservative | 0.1-0.6 |

The SEM photos indicated that treatment with both the aluminum chlorohydrate formulation and the zinc lactate formulation resulting in clogging of the apocrine ducts and hair sheaths, indicating both formulations would be effective for offering reduction of sweating.

### Example 2

The following are antiperspirant compositions according to the invention.

An emulsion roll-on formulation, which exhibits excellent physical stability and application properties, is shown in Tables 6a and 6b.

**TABLE 6a**

| INGREDIENT | FUNCTION | % w/w |
|---|---|---|
| A: Zinc Lactate | Antiperspirant Agent | 3.50 |
| B: Steareth-21 | Surfactant | 2.00 |
| C: Steareth-2 | Surfactant | 2.00 |
| D: Steareth-5 Stearate | Skin Conditioning | 1.00 |
| E: Cyclomethicone (and) PPG-15 Stearyl Ether | Skin Conditioning | 5.00 |
| F: Deionized Water | Solvent | 75.5 |
| G:Absorbents | Absorbents | 6.00 |
| H: Fragrance | Perfume | q.s |

**TABLE 6b**

| INGREDIENT | FUNCTION | % w/w |
|---|---|---|
| A: Zinc Phenolsulfonate | Antiperspirant Agent | 5.00 |
| B: Steareth-21 | Surfactant | 2.00 |
| C: Steareth-2 | Surfactant | 2.00 |
| D: Steareth-5 Stearate | Skin Conditioning | 1.00 |
| E: Cyclomethicone (and) PPG-15 Stearyl Ether | Skin Conditioning | 5.00 |
| F: Deionized Water | Solvent | 75.5 |
| G:Absorbents | Absorbents | 6.00 |
| H: Fragrance | Perfume | q.s |

### Procedure

1. Combine B, C, D and E and heat to 700C
2. Heat A, and F separately at 700C
3. Add B/C/D/E to F with agitation
4. Homogenize the mixture for 1-3 min
5. Cool to 35°C with continuous agitation
6. Add G to emulsion slowly with agitation
7. Homogenize the mixture again for 1-3 min
8. Add I
9. Fill into suitable containers

The composition of the invention may also be a clear water-in-oil roll-on, which demonstrates superior aesthetics and leave no residue or deposit on the skin after application. Clarity is achieved simply by following the room temperature order of addition specified. An example of such a composition according to the invention is shown in Tables 7a and 7b.

**TABLE 7a**

| INGREDIENT | FUNCTION | % w/w |
|---|---|---|
| A: Zinc Lactate | Antiperspirant Agent | 3.50 |
| B: Deionized Water | Solvent | 8.75 |
| C: Dipropylene Glycol | Solvent | 3.00 |
| D: PEG-7 Glyceryl Cocoate | Skin Conditioning | 18.20 |
| E: Cyclomethicone (and) Dimethicone Copolyol | Skin Conditioning | 45.50 |
| F: Cetearyl Octanoate | Surfactant | 3.20 |
| G: Polysorbate 20 | Surfactant | 1.00 |
| H: Deionized Water | Solvent | 4.10 |
| I: Isopropyl Myristate | Skin Conditioning | 1.00 |
| J: Absorbents | Absorbents | 6.00 |
| K: Fragrance | Perfume | 0.75 |

**TABLE 7b**

| INGREDIENT | FUNCTION | % w/w |
|---|---|---|
| A: Zinc Phenolsulfonate | Antiperspirant Agent | 5.00 |
| B: Deionized Water | Solvent | 8.75 |
| C: Dipropylene Glycol | Solvent | 3.00 |
| D: PEG-7 Glyceryl Cocoate | Skin Conditioning | 18.20 |
| E: Cyclomethicone (and) Dimethicone Copolyol | Skin Conditioning | 45.50 |
| F: Cetearyl Octanoate | Surfactant | 3.20 |
| G: Polysorbate 20 | Surfactant | 1.00 |
| H: Deionized Water | Solvent | 4.10 |
| I: Isopropyl Myristate | Skin Conditioning | 1.00 |
| J: Absorbents | Absorbents | 6.00 |
| K: Fragrance | Perfume | 0.75 |

### Procedure

1. Combine A, B, C, and with overhead mixing (medium agitation)
2. Slowly add D. Mix well
3. Slowly add E. Mix well
4. Slowly add F. Mix well
5. Premix G and H. Slowly add to the main batch
6. Slowly add J. Mix well
7. Premix I and K. Slowly add to the main batch. Mix until clear
8. Pour into appropriate containers

The composition of the invention may also be a foam. Accordingly, the liquid vehicles described above may contain dispersions of gas in the liquid phase. The gas globules may be of any size, from colloidal to macroscopic, as in soap bubbles. Typical liquid foams are those used in shaving creams, etc.

## Claims

1. An antiperspirant composition comprising at least 5% by weight of zinc phenolsulphonate and/or at least 3.5% by weight of zinc lactate, wherein said composition is substantially free of aluminum salts.

2. The composition of claim 1 further comprising a water absorbing agent.

3. The composition of claim 2, wherein the water absorbing agent is selected from the group consisting of silicas, cotton powder, bamboo silk and zeolites.

4. The composition of claim 1 wherein the antiperspirant composition comprises zinc phenolsulphonate.

5. The composition of claim 1 wherein the antiperspirant composition comprises zinc lactate.

6. The composition of claim 1 wherein the antiperspirant composition comprises a combination of zinc phenolsulphonate and zinc lactate.

7. The composition of claim 1 further comprising acrylates/octylacrylamide copolymer.

8. The composition of claim 1 further comprising an antibacterial ingredients selected from the group comprising 1,6-di-(4-chlorophenylbiguanido) hexane, 2,4,4'-trichlorocarbanilide, quaternary ammonium compounds, oil of cloves, mint oil, thyme oil, triethyl citrate, 3,7,11-trimethyl-2,6,10-dodecatrien-1-ol, ethylhexyl glycerol ether and polyglyceryl-3-caprylate.

9. Use of a composition as an antiperspirant, wherein said composition comprises at least 3.5% by weight of zinc phenolsulphonate and/or zinc lactate, wherein said composition is substantially free of aluminum salts.

## Patentansprüche

1. Antiperspirantzusammensetzung, umfassend mindestens 5 Gew.-% Zinkphenolsulfonat und/oder mindestens 3,5 Gew.-% Zinklactat, wobei die Zusammensetzung weitgehend frei von Aluminiumsalzen ist.

2. Zusammensetzung nach Anspruch 1, ferner umfassend ein wasserabsorbierendes Mittel.

3. Zusammensetzung nach Anspruch 2, wobei das wasserabsorbierende Mittel aus der Gruppe bestehend aus Siliciumdioxiden, Baumwollpulver, Bambusseide und Zeolithen ausgewählt ist.

4. Zusammensetzung nach Anspruch 1, wobei die Antiperspirantzusammensetzung Zinkphenolsulfonat umfasst.

5. Zusammensetzung nach Anspruch 1, wobei die Antiperspirantzusammensetzung Zinklactat umfasst.

6. Zusammensetzung nach Anspruch 1, wobei die Antiperspirantzusammensetzung eine Kombination von Zinkphenolsulfonat und Zinklactat umfasst.

7. Zusammensetzung nach Anspruch 1, ferner umfassend Acrylates/Octylacrylamide Copolymer.

8. Zusammensetzung nach Anspruch 1, ferner umfassend einen antibakteriellen Bestandteil aus der Gruppe umfassend 1,6-Di(4-chlorphenylbiguanido)hexan, 2,4,4'-Trichlorcarbanilid, quaternäre Ammoniumverbindungen, Nelkenöl, Minzöl, Thymianöl, Triethylcitrat, 3,7,11-Trimethyl-2,6,10-dodecatrien-1-ol, Ethylhexylglycerinether und Polyglyceryl-3-caprylat.

9. Verwendung einer Zusammensetzung als Antiperspirant, wobei die Zusammensetzung mindestens 3,5 Gew.-% Zinkphenolsulfonat und/oder Zinklactat umfasst, wobei die Zusammensetzung weitgehend frei von Aluminiumsalzen ist.

## Revendications

1. Composition d'agent antitranspirant comprenant au moins 5% en poids de phénolsulfonate de zinc et/ou au moins 3,5% en poids de lactate de zinc, ladite composition étant sensiblement exempte de sels d'aluminium.

2. Composition selon la revendication 1, comprenant en outre un agent d'absorption de l'eau.

3. Composition selon la revendication 2, l'agent d'absorption de l'eau étant choisi dans le groupe constitué par les silices, la poudre de coton, la soie de bambou et les zéolites.

4. Composition selon la revendication 1, la composition d'agent antitranspirant comprenant du phénolsulfonate de zinc.

5. Composition selon la revendication 1, la composition d'agent antitranspirant comprenant du lactate de zinc.

6. Composition selon la revendication 1, la composition d'agent antitranspirant comprenant une combinaison de phénolsulfonate de zinc et de lactate de zinc.

7. Composition selon la revendication 1, comprenant en outre un copolymère d'acrylates-octylacrylamide.

8. Composition selon la revendication 1, comprenant en outre un ingrédient antibactérien choisi dans le groupe comprenant le 1,6-di-(4-chlorophénylbiguanidino)hexane, le 2,4,4'-trichlorocarbanilide, les composés d'ammonium quaternaire, l'essence de girofle, l'essence de menthe, l'essence de thym, le citrate de triéthyle, le 3,7,11-triméthyl-2,6,10-dodécatrién-1-ol, l'éther d'éthylhexylglycérol et le poly(caprylate de 3-glycéryle).

9. Utilisation d'une composition en tant qu'agent antitranspirant, ladite composition comprenant au moins 3,5% en poids de phénolsulfonate de zinc et/ou de lactate de zinc, ladite composition étant sensiblement exempte de sels d'aluminium.
